(19) Europäisches Patentamt
European Patent Office
Office européen des brevets

(11) EP 3 848 936 A1

(12) EUROPEAN PATENT APPLICATION
published in accordance with Art. 153(4) EPC

(43) Date of publication:
**14.07.2021 Bulletin 2021/28**

(21) Application number: **19857095.4**

(22) Date of filing: **04.09.2019**

(51) Int Cl.:
***G16B 40/00*** (2019.01) ***G16B 30/10*** (2019.01)

(86) International application number:
**PCT/KR2019/011410**

(87) International publication number:
**WO 2020/050627 (12.03.2020 Gazette 2020/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **05.09.2018 US 201862727121 P**
**03.09.2019 KR 20190109117**

(71) Applicant: **Chunlab, Inc.**
**Seoul 06194 (KR)**

(72) Inventors:
- **WILLIAMS, Mauricio Antonio Chalita**
  **Seoul 08826 (KR)**
- **YOON, Seok-Hwan**
  **Seoul 08848 (KR)**
- **HA, Sung-Min**
  **Yongin-si, Gyeonggi-do 16813 (KR)**

(74) Representative: **Grünecker Patent- und Rechtsanwälte PartG mbB**
**Leopoldstraße 4**
**80802 München (DE)**

# (54) METHOD FOR IDENTIFYING AND CLASSIFYING SAMPLE MICROORGANISMS

(57) The present invention relates to a method for identifying and classifying microorganisms included in a sample by using an exact k-mer matching algorithm and a bacterial core gene and, preferably, can more quickly and more accurately analyze the taxonomic composition of a metagenomic sample without bias.

**Fig. 1**

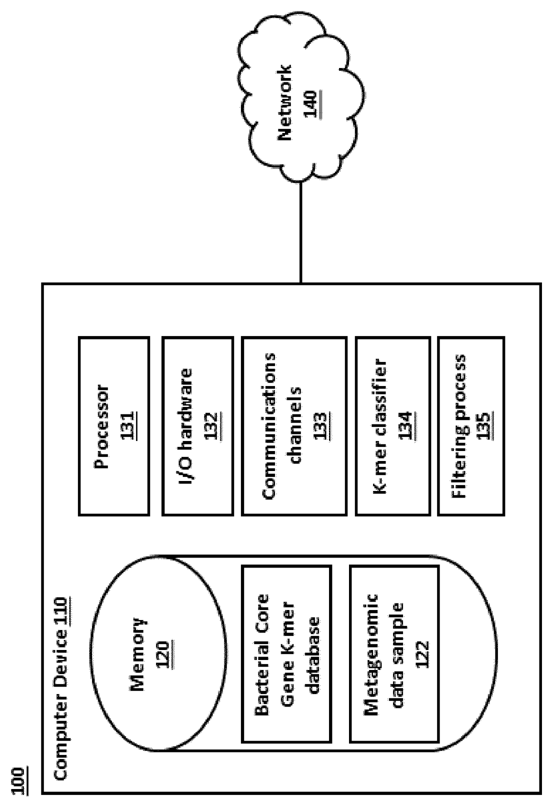


EP 3 848 936 A1

<110> ChunLab, Inc.

<120> taxonomy profiling method for microorganism in sample

<130> OPP20192300KR

<150> US 62/727,121
<151> 2018-09-05

<150> KR 10-2019-0109117
<151> 2019-09-03

<160> 8

<170> KoPatentIn 3.0

<210> 1
<211> 14
<212> DNA
<213> Artificial Sequence

<220>
<223> query read

<400> 1
cgacgcaacc cgtt 14

<210> 2
<211> 12
<212> DNA
<213> Artificial Sequence

<220>
<223> K-mer 1

<400> 2
cgagcgcaac cc 12

<210> 3
<211> 12
<212> DNA
<213> Artificial Sequence

<220>
<223> K-mer 2

<400> 3
ggagcgcaac cc 12

<210> 4
<211> 12
<212> DNA
<213> Artificial Sequence

<220>
<223> K-mer 3

```
<400>  4
agcgcaaccc gt                                                          12


<210>  5
<211>  12
<212>  DNA
<213>  Artificial Sequence

<220>
<223>  K-mer 4


<400>  5
gcgcaacccg tt                                                          12


<210>  6
<211>  23
<212>  DNA
<213>  Artificial Sequence

<220>
<223>  K-mer 5


<400>  6
cgagcgcaac cccctacgtt tag                                              23


<210>  7
<211>  24
<212>  DNA
<213>  Artificial Sequence

<220>
<223>  K-mer 6


<400>  7
cgcaacccct acgtttagtt gcta                                             24


<210>  8
<211>  24
<212>  DNA
<213>  Artificial Sequence

<220>
<223>  K-mer 7


<400>  8
ctacgtttag ttgctaacag gtga                                             24
```

## Description

### Technical Field

The present invention relates to a taxonomic profiling method for microbes in a sample and a method for analysis of microbial species abundances in the sample, each method using an exact k-mer match algorithm and bacterial core genes, whereby a taxonomic composition of a metagenome sample can be analyzed faster and more accurately without bias.

### Background Art

In the last decade, it has been difficult to predict taxonomic compositions of metagenomic samples. Taxonomic classification of microbes contained in a given sample could provide much insight into roles of the microbes in environments. Analysis of databases updated with new genomes publicized annually allows more accurate and specific classification. However, this process requires an extremely large number of complicated calculations based on millions of reads from samples against thousands of reference genomes, which can be fulfilled only by use of a very large CPU clusters as a rule.

For the last few decades, taxonomic classification has been achieved through homology search (sequence alignment). This approach is useful when "the closest" match with a specific genomic read is searched for in the absence of sufficient information for a reference database. If a reference database is not available for a given species, a number of reads are not classified, making the "extract k-mer matching" approach unreliable due to insufficient information of the databases.

However, with the increase of the number of publicly available genomes, the "exact k-mer matching" approach has become sufficiently reliable in recent years. In addition, the computer capacity that has been enhanced enables the approach to be realized and thus to become useful. In contrast, a homology searching method slows down the analyzing speed of the computer due to a large number of comparisons to be performed and is inaccurate because relevant genomes have similar levels of sequence compositions. In order to avoid the inaccuracy and reduce calculation time, some homology searching methods take advantage of gene markers (sequences that only occur once within various species or genera) in reducing the number of comparisons. The method utilizing gene markers is disadvantageous in that sizes of bacterial genomes and frequencies of genes are very irregular (some species or genera include more markers than other species) and when another specie or genus is added to a reference database, calculation must be made again for the corresponding maker. When discovered in a completely different classified group, a preexisting marker can be used no more for the existing groups.

In order to acquire more accurate taxonomic profiles, all species in the taxonomic profiles must be normalized. For a metagenomic taxonomic profile using total genome database as a reference database, a normalization step contemplating genome size of each species must be included. For example, species A having a genome size of 5 Mb makes more contributions to a sample, compared to species B having a genome size of 2 Mb. In the case where a gene marker is used, the number of reads obtained by NGS (Next Generation Sequencing) must be normalized according to the size and frequency of the gene marker in the same genome.

Metagenome is a term used for the analysis of genetic materials together in a sample containing various microbes, for example, a sample taken from an environment. Recent various researches make it possible to list bacterial compositions of microbiomes in human bodies and environments through metagenome NGS data analysis based on marker genes of 16S rRNAs. In addition, active studies on metagenomic NGS data analysis using a shotgun approach are ongoing.

## Disclosure

### Technical Problem

The present invention provides a method for identification and classification of two or more microbial species in a sample faster and more accurately without bias, by analyzing a taxonomic composition with extract k-mer matching method and bacterial core genes, and a system for identification and classification of microbes in a sample.

An embodiment of the present invention provides a taxonomic profiling method by analyzing species abundance of microbes in a sample, especially a metagenomic sample, with an exact k-mer matching method and bacterial core genes.

## Technical Solution

An embodiment of the present invention relates to method of identifying and classifying microorganisms in a sample, the method comprising the step of:

providing microbial genome information obtained from the sample;
obtaining a sample k-mer dataset using the microbial genome information; and
comparing the sample k-mer dataset with a microbial taxon information-assigned reference k-mer database of reference microbial core genes to identify and classify microbes in the sample.

An additional embodiment of the present invention can obtain information on abundance of microorganisms in a sample using a method of identifying and classifying microorganisms in a sample, or more specifically provide a method comprising:

providing microbial genome information obtained from the sample,
obtaining a sample k-mer dataset using the microbial genome information,
comparing the sample k-mer dataset with a microbial taxon information-assigned reference k-mer database of reference microbial core genes to identify and classify microbes in the sample, and
obtaining abundance profile information of microbial species in the sample by calculating abundance of microorganisms in the sample.

In a specific embodiment, the method of identifying and classifying microorganisms in a sample of the present invention may perform the steps, by utilizing a computer device:

obtaining microbial genome information containing sequencing reads obtained analyzing microorganism genomic DNA from a sample through next generation sequencing (NGS),
obtaining a sample k-mer dataset for full genomes of the microorganisms in the sample using the microbial genome information by creating a k-mer dataset for each of the sequencing reads, and
comparing the sample k-mer dataset with a microbial taxon information-assigned reference k-mer database of reference microbial core genes to identify and classify the microorganisms in the sample.

In an additional embodiment of the present invention, the method of identifying and classifying microorganisms in a sample includes the reference k-mer database in which each k-mers is assigned by unique ID values classified for the microbial taxon information, and the microbial genome information containing sequencing reads obtained through next generation sequencing (NGS),
for each sequencing read of sample microbial genome,
is classified by unique ID values and is assigned to individual k-mers in the reference k-mer database; the sample microbial genome information includes sequencing reads obtained by next generation sequencing (NGS); and
for individual sequencing reads of the sample microbial genome,

(i) creating a k-mer dataset including one or more k-mers and comparing with the reference k-mer database of reference micr0bial core genes (bacterial core gene) to select a k-mer whose nucleotide sequence is exactly matched, from the reference k-mer database,
(ii) obtaining unique ID information assigned to the selected k-mer,
(iii) selecting an ID as a unique ID for a sequencing read if a unique ID list includes one unique ID or all identical unique IDs, or selecting a unique ID corresponding to the least common ancestor (LCA) if a unique ID list includes two or more different unique IDs, using list information including one or more unique IDs obtained for the sequencing reads,
(iv) combining taxon information of the unique IDs corresponding to the taxonomic levels assigned to individual sequencing reads, and

the microbe in the sample is identified and classified by generating a full unique ID list with collecting the unique IDs corresponding to the taxonomic levels obtained for the individual sequencing reads for entire sequencing reads included in the sample microbial genome.
In addition, the method for obtaining taxonomic profiling information or an abundance of microbes in a sample, or more specifically an abundance profile information of microbial species in a sample, comprises the steps of:

providing microbial genome information obtained from the sample;

obtaining a sample k-mer dataset using the microbial genome information; and
comparing the sample k-mer dataset with a microbial taxon information-assigned reference k-mer database of reference microbial core,
wherein the sample microbial genome information includes sequencing reads obtained by next generation sequencing (NGS), and
for individual sequencing reads of the sample microbial genome,

(i) creating a k-mer dataset including one or more k-mers and comparing with the reference k-mer database of reference microbial core genes to select a k-mer whose nucleotide sequence is exactly matched, from the reference k-mer database,
(ii) obtaining unique ID information assigned to the selected k-mer,
(iii) selecting an ID as a unique ID for a sequencing read if a unique ID list includes one unique ID or all identical unique IDs, or selecting a unique ID corresponding to the least common ancestor (LCA) if a unique ID list includes two or more different unique IDs, using list information including one or more unique IDs obtained for the sequencing reads,
(iv) combining taxon information of the unique IDs corresponding to the taxonomic levels assigned to individual sequencing reads,

generating an entire unique ID list with collecting the unique IDs corresponding to the taxonomic levels obtained for the individual sequencing reads for entire sequencing reads included in the sample microbial genome.
obtaining the number of classified reads by unique ID corresponding to the taxonomic level from the entire unique ID list for microbes in the sample, and
obtaining abundance in the sample for the microbial species or classification information corresponding to the unique IDs by dividing the number of classified reads by unique ID with a sum of the number of classified reads in the entire unique ID list.

By using the method of present invention, information about at least one selected from the group consisting of species, the lowest common ancestor of the microbial species, taxonomic classification, populations of specific species, and relative abundance of the microbes can be generated for a sample containing at least two microbial species or at least to microbial genome information, for example, a metagenome sample.

An embodiment of the present invention provides a system of identifying and classifying microorganism in a sample, the system comprising a reference k-mer database of reference microbial core genes, and a processor equipped with a k-mer extractor and a k-mer analyzer,
wherein the reference k-mer database comprises at least one k-mer generated from DNA information of at least one reference microbial core gene, and the k-mer is assigned with microbial taxon information,
wherein the k-mer extractor in the processor extracts at least one k-mer from microbial genome information obtained from the sample to generate k-mer dataset; and
wherein the k-mer analyzer in the processor selects a k-mer exactly identical in nucleic acid sequence information from the k-mers included in the reference k-mer database of reference core genes with respect to the k-mer included in the sample k-mer dataset, lists unique IDs accounting for taxon information of the selected k-mer, and identifies and classifies the microorganism in the sample, based on the taxonomic information about the selected k-mer.

Another embodiment of the present invention provides a method of obtaining abundance profile of microbial species in a sample, the system comprising: a reference k-mer database of reference microbial core genes (bacterial core genes); and a processor equipped with a k-mer extractor, a k-mer analyzer, and an abundance analyzer, wherein the k-mer extractor and the k-mer analyzer are as defined above, and the abundance analyzer is adapted to analyze a population scale of which specific species occupy in entire microorganisms of the sample and the population can be calculated in various methods.

According to an embodiment, when genome information of microbes in the sample is obtained by NGS and the taxon information is classified by unique ID values and is assigned to individual k-mers in the reference k-mer database, the abundance analyzer subjects the individual sequencing reads of the sample microbial genome to the following processes of:

(i) creating a k-mer dataset including one or more k-mers and comparing with the reference k-mer database of reference micr0bial core genes (bacterial core gene) to select a k-mer whose nucleotide sequence is exactly matched, from the reference k-mer database;
(ii) obtaining unique ID information assigned to the selected k-mer;
(iii) selecting an ID as a unique ID for a sequencing read if a unique ID list includes one unique ID or all identical unique IDs, or selecting a unique ID corresponding to the least common ancestor (LCA) if a unique ID list includes two or more different unique IDs, using list information including one or more unique IDs obtained for the sequencing reads; and

(iv) combining taxon information of the unique IDs corresponding to the taxonomic levels assigned to individual sequencing reads, and

generating an entire unique ID list with collecting the unique IDs corresponding to the taxonomic levels obtained for the individual sequencing reads for entire sequencing reads included in the sample microbial genome.

obtaining the number of classified reads by unique ID corresponding to the taxonomic level from the entire unique ID list for microbes in the sample, and

obtaining abundance in the sample for the microbial species or classification information corresponding to the unique IDs by dividing the number of classified reads by unique ID with a sum of the number of classified reads in the entire unique ID list.

Hereinafter, the present invention will be described in more detail.

The present invention relates to a method for identifying and classifying microbial species in a sample and a system for identifying and classifying microbial species in a sample, using an exact k-mer matching method and bacterial core genes.

Specifically, the method and the system for identifying and classifying the microbial species in a sample according to the present invention may comprise the steps of: providing (a) a sample k-mer dataset for a full genome of microbes in the sample, which is created by utilizing microbial genome information obtained from a sample, and (b) a taxon information-assigned reference k-mer database of reference microbial core genes; (c) comparing the k-mers in the sample k-mer dataset (a) with the k-mers in the reference k-mer database (b) according to an exact k-mer matching method to select an exactly matched k-mers; and (d) identifying and classifying the microbial species in the sample using taxon information of the selected k-mers.

The method and the system for identifying and classifying microbes according to the present invention comprises a step of (a) creating a sample k-mer dataset for a full genome of bacteria in the sample by utilizing microbial genome information obtained from the sample.

The step of creating a sample k-mer dataset may comprise (a-1) extracting full genome DNA of at least one microorganism in a test sample (genomic DNA extraction), (a-2) obtaining nucleotide sequence information by sequencing the entire genome DNA of the test microbes (sequence information analysis), (a-3) extracting at least one k-mer from the microbial genome information to create a k-mer dataset (sample k-mer dataset creation).

The sub-step (a-1) may be carried out separately and the creating step may start with the sub-step (a-2) of providing nucleotide sequence information of microbial full genomic DNA in the sample. Thus, the (a-1) genomic DNA extraction step may not be included in the method for identifying and classifying microbes according to the present prevention.

In the step of creating a sample k-mer dataset of the present invention, the sub-step of extracting full genomic DNA of at least one microbial species in a test sample is not particularly limited and may be performed in any manner known in the art for DNA extraction.

The step of creating a sample k-mer dataset of the present invention comprises the sub-step of obtaining nucleotide sequence information by sequencing the genomic DNA of whole test microbes in the sample. The sequencing of the genomic DNA of all microbes in a sample may be carried out using any DNA sequencing method known in the art.

For example, the microbiome is the genome information of all the microbes in a sample and can be obtained using various methods, for examples, NGS or shotgun sequencing method. Input nucleotide data of a metagenome sample to be analyzed may be obtained by sequencing DNA of the metagenome sample by massively parallel sequencing methods such as such as shotgun metagenome sequencing method or next-generation sequencing method.

When the genome sequence information is analyzed with next-generation sequencing (NGS), the microbial genome information may include sequencing reads obtained by NGS.

Shotgun metagenome sequencing is a technique of randomly fragmenting DNA into many small pieces. Shotgun metagenome sequencing can extract comprehensively sample all genes in all organisms present in a given community and allows the evaluation of bacterial diversity and the detection of the abundance thereof in various environments. Shotgun metagenome sequencing also advantageously provides a means to study unculturable microorganisms that are otherwise difficult or impossible to analyze.

The step of creating a sample k-mer dataset of the present invention may comprise the sub-step (a-3) of extracting at least one k-mer from the microbial genome information to create a k-mer dataset (sample k-mer dataset creation).

In an embodiment, the microbial genome information includes sequencing reads obtained by next-generation sequencing (NGS). The k-mer dataset for entire bacterial genomes in a sample can be created by fragmenting the individual sequencing reads into k-mer-long letter strings the fragmenting site on each of the sequencing reads shifting by one base for each fragment, using a computer device. The creation of the sample k-mer dataset can be performed using a k-mer extractor. An exemplary k-mer extractor may be a JELLYFISH program, but is not limited thereto. JELLYFISH is a command-line program that counts k-mers in an input FASTA file.

In an embodiment of the present invention, the test sample may contain at least one microbial species and preferably at least two microbial species. More preferably, the test sample may be a metagenomic sample. Metagenome is defined as a collection of all genomes of microbes present in a given natural environment and is a generic term referring to a clone including genomes or genes extracted from an environment sample.

Generally, the term "k-mer" means a polynucleotide fragment composed of K as the number of nucleotides. The k-mer or k-mer fragment of the bacterial core gene according to the present invention refers to a polynucleotide sequence which is fragmented from a bacterial core gene in each bacterial species and has a length of "k" nucleotides. When a sequence is given, the term also refers to a collection of all possible subsequences, each being a k-mer long.

In the method described herein, at least one k-mer fragment sequence is created from the full genome sequence information of microbes present in a sample and exact matching is made between the k-mer fragment database created from the metagenome sample and k-mer sequences of a reference bacterial core gene, whereby the microbes contained in the sample can be identified and classified.

For example, the "AGCTCT" sequence can be divided into the 3-nt subsequences "AGC", "GCT", "CTC", and "TCT". These subsequences are each k-mer wherein k is 3. K-mers may or may not be overlapped.

When provided using next-generation sequencing (NGS), the microbial genome information contains sequencing reads obtained by NGS. The k-mer is preferably shorter than the sequencing reads. Generally, the term "sequencing read" (also referred to as "read" or "query sequence") means a nucleotide sequence inferred from a nucleic acid molecule.

In addition, sequencing reads obtained by general sequencing analysis may be 50 nucleotides (nt) or higher, 60 nt, 70 nt or higher, 80 nt or higher, 90 nt or higher, or 100 nt or higher. The upper limit of the length is not particularly limited, but may be 5,000 nt or less, 4,000nt or less, 3,000 nt or less, 2,000 nt or less, 1000 nt or less, 900 nt or less, 800 nt or less, 700 nt or less, 600 nt or less, or 500 nt or less. The sequencing reads may range in length between the upper limit and the lower limit. For example, a sequencing read may range in length from 50 to 5,000 nt, from 50 to 4,000 nt, from 50 to 3,000nt, from 50 to 2,000nt, from 50 to 1,500nt, from 50 to 1,000nt, from 50 to 900nt, from 50 to 800nt, from 50 to 700nt, from 50 to 600nt, from 50 to 500nt, from 60 to 5,000 nt, from 60 to 4,000 nt, from 60 to 3,000nt, from 60 to 2,000nt, from 60 to 1,500nt, from 60 to 1,000nt, from 60 to 900nt, from 60 to 800nt, from 60 to 700nt, from 60 to 600nt, from 60 to 500nt, from 70 to 5,000 nt, from 70 to 7,000 nt, from 70 to 3,000nt, from 70 to 2,000nt, from 70 to 1,500nt, from 70 to 1,000nt, from 70 to 900nt, from 70 to 800nt, from 70 to 700nt, from 70 to 600nt, or from 70 to 500nt.

In an exemplary embodiment, the k-mer used for taxonomically profiling metagenome in the method of the present invention may have a size or length of 10 to 100 nucleotides (nt), 10 to 90 nt, 10 to 80 nt, 10 to 70 nt, 10 to 60 nt, 10 to 50 nt, 10 to 40 nt, or 18 to 31 nt. When using a k-mer, a shorter k-mer results in fewer possible sequence combinations. Too short a k-mer sequence does not allow the provision of a sufficient number of k-mer sequences necessary for discriminating tens of thousands of known bacteria species and millions of unknown bacteria species. On the other hand, when long k-mers are used, an increased number of sequence combinations is obtained to make it possible to accurately match for specific species, but become more sensitive to sequence analysis errors and gene mutants. Thus, the sensitivity is lowered and the database increases in size because more combinations are added to the database. In addition, long sequences are calculated for consensus, requiring greater storage capacity and computer power.

In light of the characteristics of k-mer analysis, lengths of the k-mers used herein are preferably selected within the range of 10-nt to 100-nt. The lower limit allows the number of combinations that enables tens of thousands of bacterial species known up to now to be discriminated while the upper limit allows for the maintenance of sensitivity in consideration of maximal storage capacity and computer power efficiency.

The method or system for identifying and classifying microbial species in a sample according to the present invention may comprise the step (b) of building a taxon information-assigned reference k-mer database of microbial core genes (bacterial core genes), or a system including a taxon information-assigned reference k-mer database of reference microbial core genes (bacterial core genes). That is, the microbial species in a sample can be identified and classified on the basis of the microbial taxon information included in the reference k-mer database of microbial core genes, by comparing the sample k-mer dataset with the reference k-mer database of reference microbial core genes, The taxon information-assigned reference k-mer database of reference microbial core genes may be built by (b-1) obtaining nucleotide sequence information of whole microbial core genes of at least two reference microbial species and (b-2) dividing the sequence information of the reference core genes into k-mers and assigning taxon information to each k-mer.

The reference k-mer database contains any bacterial core sequence to be compared with a k-mer dataset. When a core gene of a new reference microbe is discovered, the reference k-mer database may be rebuilt therewith. In the reference k-mer database, taxonomic information is assigned to individual reference k-mer sequences which may be further given information about some known characteristics including a sample source, a taxonomic group, a specific species, an expression profile, a specific gene, a phenotype associated with possibility of disease onset, a drug resistance, or pathogenicity.

The reference k-mer database used in the present invention is built with bacterial core gene sequences and has to include at least one core gene for each bacterial genome.

For taxonomic profiling, a k-mer fragment database of reference core genes is constructed in the present invention and includes at least one k-mer fragment derived from the reference core gene wherein the taxon information is assigned to the k-mer fragment. For construction of the k-mer fragment database of the reference core gene, reference core gene information is obtained from reference microbial genome information and divided into K-mer fragments. A taxon is assigned to the k-mer fragment.

As used herein, the term "bacterial core gene" is widely defined as a homologous gene that is present as a single copy in all or most of known bacterial species. The core gene is similar to a single-copy gene and varies in number depending on the species included in the database. In detail, the bacterial core gene may exist as a single copy gene in the genome information of total reference microbes used to build the k-mer database of reference core genes.

The bacterial core gene to be used in the present invention may range in length from 100 to 4,000 bases (nucleotides, nt), for example, 110 to 4,000 nt, 120 to 4,000nt, 125 to 4,000 nt, 110 to 3,900 nt, 120 to 3,900nt, 125 to 3,900 nt, 110 to 3,800 nt, 120 to 3,800nt, or 125 to 3,800 nt. However, so long as it is possible to use taxonomic classification of microbes, any suitable length can be selected.

The bacterial core gene used in an embodiment of the present invention can be selected in consideration of the ratio of the number of unique k-mer sequences to the number of total k-mer sequences (A) and/or the ratio of the number of unique k-mer sequences to the number of distinct k-mers. Preferably, the bacterial core gene may have a (A) ratio of 40 % or more and/or a (B) ratio of 75 % or more. A longer k-mer results in greater (A) and (B).

Table 1 shows numbers of unique k-mers, distinct k-mers, and total k-mers and percentages of unique k-mers having various sizes in a k-mer database of bacterial core genes according to an embodiment of the present invention. The k-mer database of bacterial core genes for reference microbes may be altered with the addition of reference microbes and/or core genes.

[TABLE 1]

| K-MER | UNIQUE K-MER | DISTINCT K-MER | TOTAL K-MER | % UNIQUE/ TOTAL(A) | % UNIQUE/ DISTINCT(B) |
|---|---|---|---|---|---|
| 18-MER | 363,525,154 | 468,899,565 | 853,569,804 | 42.59% | 77.53% |
| 19-MER | 399,637,903 | 500,437,226 | 852,676,437 | 46.87% | 79.86% |
| 20-MER | 427,712,212 | 525,216,354 | 851,783,073 | 50.21% | 81.44% |
| 21-MER | 451,477,133 | 546,437,706 | 850,889,713 | 53.06% | 82.62% |
| 22-MER | 471,689,977 | 564,065,270 | 849,996,360 | 55.49% | 83.62% |
| 23-MER | 489,970,811 | 579,921,994 | 849,103,008 | 57.70% | 84.49% |
| 24-MER | 507,032,210 | 594,672,711 | 848,209,657 | 59.78% | 85.26% |
| 25-MER | 521,868,962 | 607,160,148 | 847,316,310 | 61.59% | 85.95% |
| 26-MER | 535,633,987 | 618,661,812 | 846,422,966 | 63.28% | 86.58% |
| 27-MER | 548,687,214 | 629,527,246 | 845,529,622 | 64.89% | 87.16% |
| 28-MER | 559,987,132 | 638,730,122 | 844,636,281 | 66.30% | 87.67% |
| 29-MER | 570,565,403 | 647,290,834 | 843,742,946 | 67.62% | 88.15% |
| 30-MER | 580,667,503 | 655,437,601 | 842,849,612 | 68.89% | 88.59% |
| 31-MER | 589,417,897 | 662,366,494 | 841,956,284 | 70.01% | 88.99% |

As used herein, the term "unique k-mer" means a k-mer sequence present as a single copy in all sequences of bacterial core genes in reference microbe population and excludes k-mer sequences that existing as two or more copies. The distinct k-mer refers to a k-mer sequence that is present as one or mor copies including repeating k-mers and unique k-mers, but is counted as one copy. In Table 1, thus, the number of distinct k-mers is a sum of the number of the unique k-mers and the number of single copies selected from repeating k-mers. The total k-mer means a sum of all single k-mers in bacterial core genes of a reference microbe population. An illustrative example is as follows:

k-mer set = {AA, AC, AC, AG, AG, AG};

Unique k-mer = {AA} = 1 k-mer;
Distinct k-mer = {AA, AC, AG} = 3 k-mers;
Total k-mer = {AA, AC, AC, AG, AG, AG} = 6 k-mers.

The k-mer is a distinctive item used in the database extracted from core genes. In the unique k-mer, corresponding k-mers mean single strains or single species. In the distinct k-mer, the k-mers except for unique k-mers, are each discovered in at least two or more strains (genomes) or two or more core genes. With respect to the k-mers that are discovered in two or more microbial genomes, when the individual genomes belong to different classification groups, the lowest common ancestor (LCA) using each classification group information is used as taxonomic information for the corresponding k-mers.

Using microbial genome information in a sample as input data, the sample k-mer dataset calculates exact k-mer matching for distinct k-mers among the three items of k-mers. The distinct k-mers including the unique k-mers are each assigned taxon information, thereby allocating taxon information lists to sequencing reads.

The use of k-mers of bacterial core genes are advantageous in that when taxonomic abundance is calculated for a given sample, the necessity of a read normalization step is removed. For total genome sequences, a large-size genome tends to provide a greater number of reads for a metagenome sample than a small-size genome. For example, species A having ten million base pairs provides 5-fold more reads for a sample per cell than species B having two million base pairs. When a metagenome profile includes ten million reads of species A and ten million reads of species B, species A and B are inferred to have one and five genomes, respectively, due to the difference of genome size therebetween although species A and B are identical in the number of reads. This condition may be true of a method using 16S rRNA sequences because a bacterial genome includes several copies of 16S rRNA. In this regard, 16S rRNA reads make different contributions to individual species in a given metagenome sample. In contrast, no normalization steps are necessary for the bacterial core gene because it is present in all or most species and exists as a single copy in all of the genomes.

In addition, the use of k-mer sequences of bacterial core gene can reduce the size of a physical storage medium necessary for storing and analyzing all metagenome samples. By way of example, a total reference genome k-mer database for 10,000 species requires a capacity of 450 gigabytes in any type of physical storage mediums whereas about 7 gigabytes are sufficient for a bacterial core gene k-mer database of the same 10,000 species. In practice, thus, the storage size is reduced by about 6,400% in a storage medium. The size reduction of storage space allows for the use of faster physical storage medium such as RAM or a solid-state drive.

The method described herein enjoys the advantage of applying an exact k-mer matching approach to a bacterial core gene for exact taxonomic profiling of metagenomes.

In order to prepare a reference k-mer database, sizes or lengths of k-mers should be selected. In Table 1, effects of k sizes on the database are shown. As defined in step (A), the core gene set is of a unique k-mer (k-mer present as a single copy in the full genome) in a given gene and thus must have a high percentage of unique k-mers. It includes a taxonomic classification system and science name list for microbial genomes for use in building a database of reference core genes.

The reference k-mer database may be produced using an algorithm or program designed to count k-mers, for example, JELLYFISH. JELLYFISH is a command-line program that counts k-mers in an input FASTA file, and utilizes an efficient hash table to store a k-mer and a corresponding unique numerical ID in the memory. A hash table is a data structure that can map keys to values, using a hash function to compute an index into an array of buckets, from which the desired value can be found. DNA k-mer sequences are stored as hash keys while unique numerical IDs are stored as values (FIG. 3).

Whenever a new k-mer occurs, a new space is allocated to the hash table and a unique numerical ID is stored. In this regard, the unique numerical ID belongs to a specific species. Positions in the taxonomy system and unique taxonomic names have large information body sizes. Thus, there are unique numerical IDs for indicating corresponding taxonomic names and individual IDs are matched to each of the microbial species included in the reference database (FIG. 4). If a previously stored k-mer is discovered again in a different DNA sequence, a LCA (Lowest Common Ancestor) ID is used instead of the unique numerical ID for a specific species (FIG. 5).

The LCA IDs are produced using a taxonomy tree. For example, when a k-mer is detected in reference sequences for E. coli and Shigella sp. The LCA ID belongs to the family taxa (Enterobacteriaceae) to which the microbes belong. Once an LCA is computed, the LCD ID replaces the value in the hash table for the corresponding k-mer. All k-mers are created as hash tables in memory and stored on the hard drive. The hash table file is also known as Kraken database. Kraken is an open-source k-mer classifier and is compatible with the JELLYFISH built-in database.

As shown in Table 4 below, the bacterial core gene in the k-mer database is advantageous in that the size of the final database file is small and the database can be allocated to faster and smaller memory such as RAM memory for execution. As a consequence, the k-mer program can run hundreds of times faster.

The k-mer database of bacterial core genes reduces the percentage of classification errors at the species level by almost half, showing how a smaller database representing the same number of species as the entire genome k-mer database can be more accurate (Table 4).

In greater detail, the step (b-1) of obtaining nucleotide sequence information of the entire bacterial core genes of at least two reference microbes can be carried out by extracting genomic DNA sequences from the reference microbes and sequencing the same, by amplifying only the core genes of the reference microbes and sequencing the same, or by extracting sequence information from a database of microbial genome sequence information.

In the case where genomic DNA sequences are extracted from the reference microbes and sequenced or only the core genes of the reference microbes are amplified and sequenced, the DNA extraction and sequencing may be carried out in the same manner as in the step (a) of obtaining a sample k-mer dataset.

In addition, when nucleotide sequence information of a bacterial core gene of a reference microbe is obtained by extracting sequence information from a database of microbial genome sequence information, UBCG bioinformatics pipeline or an alternative pipeline can be used. For example, the sequence information (input dataset) of the microbial genomic DNA of the entire sample can be searched for in and downloaded from the Sequence Read Archive of the National Center for Biotechnology Information (NCBI) using the SRA toolkit program, but without limitations thereto. For example, the bacterial core gene can be extracted from the genome of the EzBioCloud database using the UBCG pipeline.

The sub-step (b-2) may be carried out by dividing the sequence information of core genes of the entire reference microbe population into k-mers and assigning taxon information to each k-mer, thereby building a taxon information-assigned k-mer database

The reference k-mer database of the reference microbe core genes includes one or more k-mers created from the reference core gene by dividing the DNA information of the reference core genes into k-mers, wherein the k-mers may be assigned taxon information. The method of building a k-mer database using the k-mer and reference microbial core gene information may be carried out in substantially the same manner as is described for the step (a) of obtaining a sample k-mer dataset. Meanwhile there is difference in that the genome information of the entire microbes in the sample is used for creating the sample k-mer dataset in step (a) whereas the core genes of the reference microbes are used for building the reference k-mer database.

Taxon information is assigned to each of the divided k-mers to build a taxon information-assigned k-mer database. For the unique k-mer, the assignment of taxon information means the assignment of individual taxon to the corresponding species because the unique k-mer accounts for a single genome or single species. In addition, there is a case where distinct k-mers, except for unique k-mers, are found in two or more core genes present in the same genome or in two or more different genomes. When distinct k-mers are discovered in two or more core genes of the same genome, taxon information is assigned to the corresponding genome. For the distinct k-mers discovered in two or more different microbial genomes which belong to different taxonomic groups, the least common ancestor (LCA) using individual taxon information is used as taxon information for corresponding k-mers.

More specifically, the reference k-mer database of reference core genes may be built by:

extracting sequence information of reference core genes from reference microbial genome information and dividing the sequence information of the reference core gene into k-mers to obtain one or more k-mers, and
assigning a unique ID for taxon information to each of the k-mers
wherein the assignment of a unique ID for taxon information to each of the k-mers may be carried out as follows: (i) when the k-mers are unique k-mers, unique IDs of the microbial species to which the corresponding k-mers belong is assigned thereto, (ii) when the k-mers are distinct k-mers and are discovered only in one microbial species, the unique ID of the corresponding microbe is assigned thereto, and (iii) when the k-mers are distinct k-mers and are discovered in various microbial species, LCA is selected and unique IDs for corresponding taxon information are assigned to the LCA.

The taxonomic profiling method or system for microbes according to the present invention may comprise the steps of (c) comparing the k-mers in the reference k-mer database with the k mers in the sample k-mer dataset according to an exact k-mer matching approach to select an exactly matched k-mers; and (d) using taxon information of the selected k-mers to identify and classify the bacterial species in the sample.

In the taxonomic profiling method or system for microbes according to the present invention, the k-mers included in the sample k-mer dataset are compared with the k-mers included in the reference k-mer database (b) to select exactly matched k-mers.

The present invention relates to a computer system that enables accurate and efficient classification of metagenome reads by comparison with a k-mer database of bacterial core genes for metagenomic taxonomic profiling. When used, the k-mer database of bacterial core genes can provide a variety of technical effects and benefits.

By using the "exact k-mer match approach," "exact k-mer alignment approach", or "k-mer perfect match" and base sequences of bacterial core genes in combination, microbial classification can be performed faster and more accurately without bias. In this regard, among all of the k-mers generated from the input data, a search is made for k-mers that exactly match the k-mers in the database and indexes containing the taxon information of the k-mers can be listed.

Generally, "exact match" or "complete match" refers to 100% identity over the shortest length of the sequences being compared (or more than the length of two sequences if they are identical). As a rule, "sequence identity" refers to the nucleotide-to-nucleotide match of two polynucleotides.

In step (c) of comparison of k-mers and selection of exactly matched k-meres, the sample k-mer dataset is compared with the reference k-mer database to examine whether or not exactly matched k-mers are present, and if a difference is detected even at one base, they are determined to be not same. When multiple identical k-mers are found in the core genes when building the k-mer database of reference core genes, they are treated as distinct k-mers. If the k-mers exactly match the k-mers of the database, the unique IDs of the k-mers are listed for the genetic information (reads in metagenome data) of the input sample. For example, base sequences are compared between k-mer fragments (e.g., extracted k-mers) obtained from the test sample and k-mer fragments (e.g., stored k-mers) from the reference k-mer database, and only the k-mer fragments that exactly match the test k-mer fragment are selected from the reference k-mer database.

The comparison of k-mers and the selection of exact match k-mers in step (c) may be carried out using a k-mer analyzer. The k-mer analyzer may be exemplified by KRAKEN. KRAKEN is a command-line application program that performs an exact match comparison of the previously built reference k-mer database (step b) and the input test k-mer fragment dataset (step a). KRAKEN is a command-line application program that performs an exact match comparison of a database and an input data set and classifies all input reads using a taxonomic tree and the lowest common ancestor (LCA) technique. If one read shows an exact match between different species, KRAKEN selects a higher taxonomic rank for the read through the LCA technique.

For (c) the comparison of k-mers and the selection of exact match k-mers by using KRAKEN and (d) taxonomic profiling of microbes in a sample, first, a reference k-mer database (hash table) is loaded to memory at which the read (DNA sequence) nucleotide sequence portion is read from the input sample k-mer dataset and the read is then divided into k-mers to perform a search based on an exact match method, as follows. Then, KRAKEN searches the corresponding k-mers to get the corresponding values (unique IDs) from the hash table. Each of the reads obtained from the input dataset is divided into k-mers to obtain a sample k-mer dataset, and the sizes of the k-mers included in the sample k-mer dataset should be coincident with those of the k-mer in the reference database.

FIG. 6 shows an example of sequencing read classification according to the present invention. In FIG. 6, a hash table (reference k-mer database) is loaded into memory and a query read (test read of genomic sequence information in the sample microbe) should be sorted. The query read (CGAGCGCAACCCGTT) (SEQ ID NO: 1) is divided into several k-mers: {CGAGCGCAACCC (SEQ ID NO: 2), GGAGCGCAACCC (SEQ ID NO: 3), AGCGCAACCCGT (SEQ ID NO: 4)}, and GCGCAACCCGTT (SEQ ID NO: 5)}. Each k-mer has a unique numerical ID. In this regard, the related ID numbers are {5756, 2347, 1345, 1345}. The ID values account for species belonging to different genera, and the read classification is assigned to the most common taxa. In this case, the classification is made at the family level. Since a k-mer sequence is used as a main key in the hash map, a certain computation time is required for searching for such a k-mer. Kraken stores all the unique IDs of the found k-mer sequences in a file and counts the number of the selected k-mers to determine how many k-mers were found for each ID. Finally, Kraken uses the number of selected k-mers to generate results (reports) showing the number of reads for each species or higher taxa.

More specifically, when the taxon information is classified by unique ID values, and is assigned to individual k-mers in the reference k-mer database, and the sample microbial genome information includes sequencing reads obtained by next generation sequencing (NGS), the method comprises:

for individual sequencing reads of the sample microbial genome,

(i) creating a k-mer dataset including one or more k-mers and comparing with the reference k-mer database of reference microbial core genes to select a k-mer whose nucleotide sequence is exactly matched, from the reference k-mer database,
(ii) obtaining unique ID information assigned to the selected k-mer,
(iii) selecting an ID as a unique ID for a sequencing read if a unique ID list includes one unique ID or all identical unique IDs, or selecting a unique ID corresponding to the least common ancestor (LCA) if a unique ID list includes two or more different unique IDs, using list information including one or more unique IDs obtained for the sequencing reads,
(iv) combining taxon information of the unique IDs corresponding to the taxonomic levels assigned to individual sequencing reads, and

generating an entire unique ID list with collecting the unique IDs corresponding to the taxonomic levels obtained for the individual sequencing reads for entire sequencing reads included in the sample microbial genome.

In addition, the present invention provides a method for obtaining profiling information on species abundance of microbes in a sample, the method comprising the steps of:

providing microbial genome information obtained from the sample;
obtaining a sample k-mer dataset using the microbial genome information; and
comparing the sample k-mer dataset with a microbial taxon information-assigned reference k-mer database of reference microbial core genes,
wherein the microbial taxon information is classified by unique ID values and is assigned to individual k-mers in the reference k-mer database,
wherein the sample microbial genome information includes sequencing reads obtained by next generation sequencing (NGS), and
for individual sequencing reads of the sample microbial genome,

(i) creating a k-mer dataset including one or more k-mers and comparing with the reference k-mer database of reference microbial core genes to select a k-mer whose nucleotide sequence is exactly matched, from the reference k-mer database,
(ii) obtaining unique ID information assigned to the selected k-mer,
(iii) selecting an ID as a unique ID for a sequencing read if a unique ID list includes one unique ID or all identical unique IDs, or selecting a unique ID corresponding to the least common ancestor (LCA) if a unique ID list includes two or more different unique IDs, using list information including one or more unique IDs obtained for the sequencing reads,
(iv) combining taxon information of the unique IDs corresponding to the taxonomic levels assigned to individual sequencing reads, and

generating an entire unique ID list with collecting the unique IDs corresponding to the taxonomic levels obtained for the individual sequencing reads for entire sequencing reads included in the sample microbial genome,
obtaining the number of the classified reads by unique ID corresponding to the taxonomic level, from the full unique ID list for microbes in the sample, and
obtaining an abundance in the sample for the microbial species or taxon information corresponding to the unique IDs by dividing the number of the classified reads by unique ID with a sum of the number of classified reads in the full unique ID list.

The method for identification and taxonomic profiling of microbes, using the bacterial core genes and k-mer dataset according to the present invention has the following advantages.

First, compared to the "homology search"-based approach, the "exact k-mer" approach according to the present invention can perform classification faster. The reason why fast classification is possible according to the exact k-mer approach is that the "exact k-mer approach" operates on a previously obtained database, called "reference k-mer database", having substrings of the genome, and only requires determining whether exact matches of strings are present in the database. However, the conventionally known homology search approach is time consuming since it is necessary to find the insertion, deletion and mutation of DNA bases over entire lengths of reads for several genomic sequences included in the reference database.

Second, compared to using entire genome sequences, microbe taxonomic classification using the bacterial core genes according to the present invention can greatly reduce the storage capacity of the database. The average genome size of all species calculated based on the EzBioCloud database is 4 million base pairs, while the length per core gene calculated through the UBCG pipeline is 1,000 base pairs on average. Therefore, the size of the database to be processed is a very important element for the taxonomic profiling of microbes in a metagenome sample containing genomes of at least two microbes as in the present invention in light of the conditions including program execution speed, storage capacity, hardware, and the time and speed of taxonomic profiling of microbes.

Third, the genetic markers conventionally used for taxonomic classification are very diverse in frequency and size, with the taxonomic classification results varying depending on the frequency and size, and are difficult to apply to a new genome. There is thus a need for an exchange for a new criterion. The bacterial core genes according to an embodiment of the present invention can cope with all genomes more equally without bias, compared to genetic markers, because all bacterial genomes contain almost the same size core genes. Taxonomically close genomes have more similar core genes which, when used in homology search, suffer from the disadvantage of creating an inaccurate or ambiguous taxonomic profile for the sub-classification group, particularly at the species level.

The method described in an embodiment of the present invention enables metagenomic taxonomic profiling based on the comparison of exact match of the k-mer sequences associated with bacterial core genes from each species in the bacterial kingdom.

Described according to an additional embodiment of the present invention is a computer system that is configured to generate a metagenomic taxonomic profile using a bacterial core gene and a k-mer database.

In a specific embodiment, the present invention provides a system of identifying and classifying a microbe in a sample, the system comprising: (a) a reference k-mer database of reference microbial core genes; and (b) a processor equipped with a k-mer extractor and a k-mer analyzer,

wherein the reference k-mer database comprises at least one k-mer generated from DNA information of at least one reference microbial core gene, and the k-mer is assigned with microbial taxon information,

wherein the k-mer extractor in the processor extracts at least one k-mer from metagenomic information obtained from the sample to generate k-mer datase; and

wherein the k-mer analyzer in the processor selects a k-mer exactly identical in nucleic acid sequence information from the k-mers contained in the reference k-mer database of reference core genes with respect to the k-mer contained in a sample k-mer dataset, lists unique IDs accounting for taxon information of the selected k-mer, and identifies and classifies the microbe in the sample, based on the taxonomic information about the selected k-mer.

The system includes at least one processor and one or more storage devices having stored computer-executable instructions. The instructions can be executed by one or more processors and receive a set of input data containing nucleotide sequences. The input sequence is compared to a k-mer database of reference bacterial core genes which is pre-built using a k-mer analyzer. Finally, the afore-mentioned k-mer analyzer can generate a taxonomic profile for the input data set.

When applied to a sample containing two or more bacterial species, for example, a metagenome sample, the taxonomic profiling method for bacterial species in a test sample according to an embodiment of the present invention comprises the steps of comparing k-mers between the sample k-mer dataset with the reference k-mer database of reference bacterial core genes through exact k-mer match to record taxon information of a specific species identified to be an exact match between the sample k-mer dataset and the k-mer database of reference core genes and/or taxon information containing LCA information for the specific species; and using the taxon information and information about a total number of exactly matched k-mers in performing classification on a k-mer dataset for test core genes to thereby generate a taxonomic profile for the sample k-mer dataset (input dataset).

The method comprises a step of selecting a taxon of an exact k-mer match for any sequence (sequencing read) obtained from an input dataset. Specifically, the method comprises a step of determining a profile according to the number of reads classified according unique ID (taxon). In the method, a list of unique IDs (e.g., numbers or letters) corresponding to the k-mers for each sequencing read is made and a taxon is selected based on the ID values. A taxon corresponding to a unique ID is selected if the unique ID is only one while LCA is used if many unique IDs are selected. Unique ID (taxon) information classified according to individual sequencing reads for all bacterial species in the input dataset is combined to obtain a number of classified reads at a taxonomic level and to determine a taxonomic profile for a microbe in the sample,

In the taxonomic profiling method for microbes according to the present invention, when genomic DNA of the microbes in a sample is analyzed by NGS, exclusion from analysis is made of fragments smaller than the sequencing read and of the k-mers that do not show exact match between the obtained sample k-mer dataset and the reference k-mer database.

In the method according to the present invention, the final taxon for all sequences in the input dataset may or may not be subjected to an additional filtering process.

The method according to the invention may produce a final result in the form of a metagenomic taxonomy report including a total number of reads at one or more taxonomic levels. No standardization steps are required because of the bacterial core genes defined above. Thus, the report can be referred to as a metagenomic abundance report.

The metagenomic taxonomic classification method of the present invention can be executed by one or more processors, and for faster classification, the k-mer database of bacterial core genes can be transferred to a faster physical storage medium such as RAM memory.

The present invention is explained with reference to the exemplary drawings.

FIG. 1 shows an example of a computing environment (100) configured for metagenome taxonomic profiling, based on an exact k-mer match between an input sample and a k-mer database of bacterial core genes. The computer environment (100) includes a computer device (110) comprising memory (120) and at least one processor (131). Other components may include a variety of different processors and memory types. The memory (120) may be any type of physical, volatile, non-volatile, external storage devices, USB memory, SSD memory, or any type of storage devices, and may be a combination of two or more types of memory.

The computer device (110) may also comprise a mouse, a keyboard, any type of monitors, a speaker, and at least one input/output hardware (132) including any device that can be used for input/output between the computer device (110) and the user.

The computer device (110) also comprises at least one communication channel (133) that can be used to communicate with at least one additional computer system. The communication channel may be in the form of a local area network (LAN), the Internet, or a similar network configuration.

The computer device (110) also comprises some executable components (134-135). Here, the executable components may be defined as software-coded components, modules, or methods that can be executed on a computing system.

FIG. 1 shows an example of a setup of a computer system designed to generate a metagenomic taxonomic profile for a given sample by comparison with a reference k-mer database of bacterial core genes. In other settings, one or more of the components may be omitted. The exemplary setup is not intended to limit the location of one or more of the components.

The memory component 120 shown in FIG. 1 comprises a bacterial core gene k-mer database (121) containing k-mers generated from a set of bacterial core genes. The core genes may vary depending on the number of species accounted for by the core gene. In addition, the memory component (120) includes a metagenomic data sample component (122) that may include one or more files containing one or more polynucleotide sequences, each being composed of at least 50 base pairs. The file may be a FASTA format file, a FASTQ format file, or any other text-based format file including polynucleotide sequences. The file represents a sample of metagenomic data and will be compared to the bacterial core gene k-mer database (121) using the k-mer analyzer 123 together with a selective filtering process (135).

FIG. 2 is a schematic diagram of a process for comparing each k-mer sequence of query reads obtained from a metagenome data sample with a reference bacterial core gene k-mer database.

The computer reading method may be implemented on a computer-readable medium with the aid of a computer-executable program.

Another embodiment provides a computer program stored in a computer-readable storage medium, which is operated in computer to execute the steps of the computer reading method. The computer program stored on a computer readable storage medium may be combined with hardware. The computer program stored in a computer-readable storage medium is to execute each step of the computer reading method, and all steps can be executed by one program or by two or more programs, each responsible for at least one step.

Another embodiment provides a computer-readable storage medium (or recording medium) in which a computer-executable program (computer executable instructions) for executing steps of the computer readable method is stored.

## Effects of the Invention

The present invention relates to a taxonomic profiling method and system for a microbe in a metagenome sample, using an exact k-mer match algorithm and a bacterial core gene, whereby a taxonomic composition in the metagenome sample can be analyzed faster and more accurately without bias.

## Brief Description of Drawings

FIG. 1 illustrates a computing environment (100) configured for metagenomic taxonomic profiling based on exact k-mer match between an input sample and a k-mer database of bacterial core genes according to an embodiment of the present invention. The computing environment (100) includes a computer device (110) having memory (120) and at least one processor (131).

FIG. 2 illustrates an example of a process for comparing reads from a metagenome data sample according to an embodiment of the present invention, in which each k-mer sequence of query reads obtained from a metagenome data sample is compared with a reference k-mer database of bacterial core genes.

FIG. 3 shows an example of a hash table for k-mer classification according to an embodiment of the present invention, where a k-mer represents a key and the ID (numerical value) of a species is stored as a value.

FIG. 4 shows a hash table including two k-mers belonging to two different species, respectively, according to an embodiment of the present invention.

FIG. 5 shows is a hash table including two k-mers according to an embodiment of the present invention, in which one of the two k-mers belongs to both two different species (5756 and 1345) and is calculated for the lowest common ancestor (LCA), instead of storing the two ID values, at a family level (ID 930).

FIG. 6 shows a hash table allocated to memory according to an embodiment of the present invention, in which the query read (CGAGCGCAACCCGTT) should be classified and is divided into a total of 4 k-mers and the 4 k-mers are retrieved from the hash table and extracted into corresponding values (5756, 2347, 1345, 1345). To classify the read, the LCA for the k-mers is selected in which case the read will be classified as node 930 (father of the nodes).

## Mode for Invention

Hereinafter, the present invention will be described in detail by examples. However, the following examples are only intended to illustrate the invention, but not to limit the scope of the invention.

## EXAMPLE 1: Building k-mer Database of Bacterial Core Gene

Using the UBCG pipeline, 92 bacterial core genes were extracted from 9,604 genomes from the EzBioCloud database. The UBCG pipeline employs phylogenetic relation in order to identify a set of core genes, which are single copies in genomes.

In brief, the method for identifying a set of bacterial core genes and the obtained data was applied to the extraction and confirmation of core genes, based on the contents of the UBCG paper (Seong-In Na et al., Journal of Microbiology (2018) Vol. 56, No.4, pp280-285). In the method of this paper, many publicized microbial genome data were analyzed and 92 genes that individual microbes have respective single copies were selected. Using HMM (Hidden Markov Model) of gene sequences corresponding to individual genes, gene sequence pattern profiles were made. The corresponding gene sequences were extracted and identified using a searching program using the gene sequence pattern profiles, such as HMMER.

The bacterial core genes were used to build a k-mer database with a JELLYFISH program. JELLYFISH is a command-line application program that counts k-mers in an input FASTA file. In this Example, k = 26.

Using JELLYFISH, a k-mer database with a 26-mer length was produced from the bacterial core gene, and the reference k-mer database thus obtained contained 87% of unique k-mers and a total size of 6.4 GB.

Analysis results of the reference k-mer database obtained in this Example are summarized in Table 2, below. Table 2 shows the number of unique k-mers, the number of distinct k-mers, the total number of k-mers, and the percentage of unique k-mers having various sizes in the k-mer database of bacterial core genes.

[TABLE 2]

| K-MER | UNIQUE K-MER | DISTINCT K-MER | TOTAL K-MER | % UNIQUE / TOTAL (A) | % UNIQUE / DISTINCT (B) |
|---|---|---|---|---|---|
| 18-MER | 363,525,154 | 468,899,565 | 853,569,804 | 42.59% | 77.53% |
| 19-MER | 399,637,903 | 500,437,226 | 852,676,437 | 46.87% | 79.86% |
| 20-MER | 427,712,212 | 525,216,354 | 851,783,073 | 50.21 % | 81.44% |
| 21-MER | 451,477,133 | 546,437,706 | 850,889,713 | 53.06% | 82.62% |
| 22-MER | 471,689,977 | 564,065,270 | 849,996,360 | 55.49% | 83.62% |
| 23-MER | 489,970,811 | 579,921,994 | 849,103,008 | 57.70% | 84.49% |
| 24-MER | 507,032,210 | 594,672,711 | 848,209,657 | 59.78% | 85.26% |
| 25-MER | 521,868,962 | 607,160,148 | 847,316,310 | 61.59% | 85.95% |
| 26-MER | 535,633,987 | 618,661,812 | 846,422,966 | 63.28% | 86.58% |
| 27-MER | 548,687,214 | 629,527,246 | 845,529,622 | 64.89% | 87.16% |
| 28-MER | 559,987,132 | 638,730,122 | 844,636,281 | 66.30% | 87.67% |
| 29-MER | 570,565,403 | 647,290,834 | 843,742,946 | 67.62% | 88.15% |

(continued)

| K-MER | UNIQUE K-MER | DISTINCT K-MER | TOTAL K-MER | % UNIQUE / TOTAL (A) | % UNIQUE / DISTINCT (B) |
|---|---|---|---|---|---|
| 30-MER | 580,667,503 | 655,437,601 | 842,849,612 | 68.89% | 88.59% |
| 31-MER | 589,417,897 | 662,366,494 | 841,956,284 | 70.01% | 88.99% |

**COMPARATIVE EXAMPLE 1: Building k-mer Database for Entire Bacterial Genome**

Another reference k-mer database was built in order to confirm the efficiency of employing bacterial core genes in a reference k-mer database.

In this experiment, the k-mer database was built in the same procedure as in Example, except for using the full genome sequence. The k-mer database for entire genomes contemplated the same species as in the k-mer database of bacterial core genes.

JELLYFISH generated a k-mer database having a 26-mer length from entire bacterial genomes and the k-mer database has a total size of 353.11 GB, which is about 55 times as large as the file size of Example 1.

**EXAMPLE 2: Evaluation of Analysis Error Rate**

2-1: Experimental sample

A previously published synthesized metagenome input file was used to verify the classification method according to the present invention. The taxonomy and approximate abundance for the synthetic dataset are described in J Basic Microbiol by Laskar F et al. 2018 Feb; 58 (2): 101-119, "Diversity of methanogenic archaea in freshwater sediments of lacustrine ecosystems."

2-2: Classification of sample microbe using reference k-mer database

The sample metagenome input files in 2-1 were sorted by the KRAKEN program using the reference k-mer database of reference bacterial core genes in Example 1 and the reference k-mer database of entire bacterial genome in Comparative Example 1.

For the reference k-mer database of small-size bacterial core genes obtained in Example 1, the database was allocated to RAM memory so that the KRAKEN program could access the database faster. It took about 9 sec to sort 296,514 reads from the input dataset.

Using the K-mer analyzer KRAKEN program, the sample k-mer dataset was compared to the k-mer database of reference bacterial core genes. KRAKEN, which is a command-line application program that performs exact match comparison between a database and an input data set, classifies all input reads using a taxonomic tree and the lowest common ancestor (LCA) technique. Through the LCA technique, KRAKEN selects a higher taxonomic rank for a read if the read shows an exact match with a different species.

The reference k-mer database of entire genomes obtained in Comparative Example 1 could not be allocated to RAM memory because of the size thereof and was instead stored on a standard hard drive. The microbe classification took 47 min, which is about 218 times longer than that for the bacterial core gene k-mer database obtained in Example 1. An additional step had to be performed because the reference k-mer database of entire genomes contained the entire genomic sequences and not all genomes were identical in size. That is, the ratio predicted using the reference k-mer database of entire genomes should be normalized using the average genome size for each species.

Ratios of classified reads for each species in the sample of Example 2-1, obtained using the reference k-mer database of the bacterial core gene built in Example 1 and the reference k-mer database of entire genomes built in Comparative Example 1, and the previously published ratios for the input dataset are shown in Table 2.

2-3: Test for analysis error rate

Analysis error rates according to the classification method using the reference k-mer database of bacterial core genes and the reference k-mer database of entire genomes were calculated by the following Equation 1, and the results are expressed as percentages of the analysis error in Table 3.

[Equation 1]

$$\%error = \frac{|\text{Predicted Abundance} - \text{Expected Abundance}|}{\text{Expected Abundance}}$$

As used in Equation 1, the term "predicted abundance" refers to a percentage predicted for given species and the term "expected abundance" means true abundance of the species existing in a sample.

In Table 2 below, the error rate is a value obtained by dividing the absolute value [Real Expected Abundance] - [(core gene k-mer]/(full genome K-mer)] by [Real Expected Abundance]. As is understood from the data of Table 2, the analysis error rate of the k-mer database of core genes according to Example 1 is lower than that of the k-mer database of entire genomes according to Comparative Example 1.

[TABLE 3]

| Taxon Name | Real Expecte d Abundan ce | Core gene K-mer | Full Genome K-mer | Core gene K-mer error rate | Full Genome K-mer error rate |
|---|---|---|---|---|---|
| Acidobacterium capsulatum | 2.61% | 2.60% | 2.53% | 0.003703998 | 0.029779365 |
| Salinispora arenicola | 0.28% | 0.37% | 0.41% | 0.321935006 | 0.46263524 |
| Salinispora tropica | 0.34% | 0.42% | 0.01% | 0.217315684 | 0.969103133 |
| Hydrogenobaculum sp. Y04AAS 1 | 2.01% | 2.15% | 1.87% | 0.070773645 | 0.069595213 |
| Persephonella marina EX-H1 | 4.74% | 4.89% | 4.75% | 0.030721803 | 0.001063051 |
| Sulfurihydrogenibium | 4.73% | 4.41% | 4.29% | 0.065836374 | 0.093074038 |
| sp. YO3AOP1 | | | | | |
| Sulfurihydrogenibium yellowstonense SS-5 | 1.55% | 1.68% | 1.37% | 0.082719243 | 0.119185568 |
| Bacteroides thetaiotaomicron | 1.70% | 1.71% | 1.64% | 0.006405815 | 0.038629334 |
| Bacteroides vulgatus | 1.12% | 1.10% | 1.07% | 0.018585894 | 0.040802891 |
| Porphyromonas gingivalis | 0.95% | 0.94% | 0.96% | 0.013598099 | 0.017745605 |
| Chlorobium limicola | 2.62% | 2.70% | 2.50% | 0.03059816 | 0.047704591 |
| Chlorobium phaeobacteroides | 2.59% | 2.30% | 2.48% | 0.114344925 | 0.043164492 |
| Chlorobium phaeovibrioides | 2.75% | 3.01% | 2.59% | 0.094744587 | 0.057192421 |
| Chlorobium tepidum | 2.61% | 2.29% | 2.52% | 0.120027611 | 0.031613641 |
| Pelodictyon phaeoclathratiforme | 1.45% | 1.57% | 1.40% | 0.078135155 | 0.035034472 |
| Chloroflexus aurantiacus J-10-fl | 0.98% | 0.99% | 0.95% | 0.008735118 | 0.037170045 |
| Herpetosiphon aurantiacus | 0.95% | 1.06% | 0.98% | 0.109358005 | 0.025605937 |
| Nostoc sp. PCC 7120 | 1.45% | 1.46% | 0.65% | 0.010607535 | 0.552470916 |
| Deinococcus radiodurans R1 | 0.40% | 0.37% | 0.48% | 0.053316155 | 0.219000116 |
| Dictyoglomus turgidum | 3.49% | 3.38% | 3.23% | 0.033483335 | 0.075514114 |
| Caldicellulosiruptor bescii | 1.76% | 1.67% | 1.75% | 0.046942124 | 0.000804692 |
| Caldicellulosiruptor saccharolyticus | 3.32% | 3.35% | 3.19% | 0.00913369 | 0.037806367 |
| Clostridium thermocellum | 2.15% | 2.04% | 2.09% | 0.050465947 | 0.026434142 |
| Enterococcus faecalis | 2.62% | 2.68% | 2.74% | 0.022407428 | 0.047589863 |

(continued)

| Taxon Name | Real Expecte d Abundan ce | Core gene K-mer | Full Genome K-mer | Core gene K-mer error rate | Full Genome K-mer error rate |
|---|---|---|---|---|---|
| Thermoanaerobacter pseudethanolicus | 2.14% | 1.67% | 0.95% | 0.222257952 | 0.556302212 |
| Fusobacterium nucleatum nucleatum | 1.27% | 1.30% | 1.40% | 0.020530105 | 0.104966976 |
| Gemmatimonas aurantiaca | 2.31% | 2.38% | 2.24% | 0.031041127 | 0.029920626 |
| Rhodopirellula baltica | 6.63% | 6.78% | 6.44% | 0.023012848 | 0.028457219 |
| Ruegeria pomeroyi | 0.28% | 0.24% | 0.34% | 0.157440662 | 0.208997792 |
| Sulfitobactersp. EE-36 | 5.26% | 5.46% | 5.69% | 0.038667238 | 0.082593424 |
| Zymomonas mobilis | 0.91% | 0.87% | 0.86% | 0.04369714 | 0.054125998 |
| Bordetella bronchiseptica | 0.34% | 0.33% | 0.32% | 0.020482807 | 0.060828604 |
| Burkholderia xenovorans LB400 | 0.32% | 0.36% | 0.34% | 0.136167936 | 0.058059438 |
| Nitrosomonas europaea | 6.24% | 6.36% | 6.33% | 0.019731391 | 0.013962519 |
| Desulfovibrio piger | 1.17% | 1.19% | 1.17% | 0.019765545 | 0.001375093 |
| Desulfovibrio vulgaris DP4 | 0.39% | 0.44% | 0.37% | 0.132561778 | 0.057134065 |
| Geobacter sulfurreducens PCA | 1.07% | 1.08% | 1.02% | 0.013517977 | 0.04804011 |
| Wolinella succinogenes | 1.61% | 1.56% | 1.54% | 0.034026849 | 0.042944484 |
| Shewanella baltica OS185 | 1.10% | 1.18% | 0.01% | 0.073049715 | 0.992845217 |
| Treponema denticola | 3.77% | 3.66% | 3.47% | 0.028622183 | 0.080175449 |
| Thermus thermophilus HB8 | 0.14% | 0.26% | 0.00% | 0.812069076 | 0.970243794 |
| Thermotoga neapolitana DSM 4359 | 5.13% | 5.04% | 5.72% | 0.0181556 | 0.11369714 |
| Thermotoga petrophila RKU-1 | 2.11% | 2.60% | 3.67% | 0.232657051 | 0.741405345 |
| Thermotoga sp. RQ2 | 6.55% | 6.54% | 6.79% | 0.002085499 | 0.036382678 |
| Akkermansia muciniphila | 1.57% | 1.52% | 1.51% | 0.03490343 | 0.040596053 |

2-4: Analysis of database for Bray-Curtis similarity distance

The reference k-mer database of bacterial core genes in Example 1 and the reference k-mer database of entire bacterial genomes in Comparative Example 1 were evaluated for Bray-Curtis similarity index.

The Bray-Curtis similarity index, also known as the Bray-Curtis distance, is based on the composition of the species levels found in both samples, and is calculated as follows: a sum of the numbers of the fewest species commonly found in both the two species is multiplied by 2 and then is divided by a sum of the numbers of the species in each species, and the resulting value is subtracted from 1. The value calculated by the Bray-Curtis distance method indicates more dissimilarity between the samples as it is closer to 1 and more similarity therebetween as it is closer to 0.

In Table 4, calculated Bray-Curtis similarity indices and known abundance are compared between the reference k-mer database of bacterial core genes in Example 1 and the reference k-mer database of entire bacterial genomes in Comparative Example 1, showing that the taxonomic profile of the reference k-er database of core genes is closer to known facts.

[TABLE 4]

| ***Assortment*** | **Bray-Curtis distance Similar (0) <-> (1) Dissimilar** |
|---|---|
| Known abundance <-> Core gene k-mer database | 0.024289264 |

(continued)

| *Assortment* | Bray-Curtis distance Similar (0) <-> (1) Dissimilar |
|---|---|
| Known abundance <-> Full Genome k-mer database | 0.050269565 |

2-5: Summary of classification using database

In this Example, previously published synthetic metagenome input files were classified using the reference k-mer database of bacterial core genes in Example 1 and the reference k-mer database of entire bacterial genomes in Comparative Example 1, and the results are summarized in Table 5, below.

In Table 2, the error rate is a unitless value obtained by dividing the absolute value of [Real Expected Abundance] - [(core gene k-mer]/(full genome K-mer)] by [Real Expected Abundance], accounting for a proportional difference from a real expected value. In Table 5, the total error is a sum of error rates for each method (Core gene k-mer / Full genome k-mer) and the average error is an average value.

[TABLE 5]

| *Comparative Explanation* | Core gene k-mer database | Full genome k-mer database |
|---|---|---|
| Database total size (gigabytes) | 6.4 | 353.11 |
| Synthetic sample species level total error | 3.728339246 | 7.401773488 |
| Synthetic sample species level average error | 0.082851983 | 0.164483855 |
| Synthetic sample profiling time (seconds) | 13 | 2840 |

As understood from data of Table 5, the bacterial core genes in the k-mer database according to Example 1 have the advantage of occupying small sizes in the final database which can be consequently allocated to faster and smaller memory such as RAM memory, leading to running the classification program hundreds of times faster.

In addition, the reference k-mer database of bacterial core gene reduced the percentage of classification errors at the species level by almost half, demonstrating that the database smaller in size can provide more accurate classification results while exhibiting the same number of species as in the entire genomic k-mer database.

**EXAMPLE 3: Accuracy Test of Microbe Classification**

3-1: Experimental sample

This experiment was performed to evaluate the accuracy of the metagenomic taxonomic classification using the k-mer database of bacterial core genes.

In this experiment, a test was made to determine whether the reference k-mer dataset of core genes according to the present invention or the reference k-mer dataset of entire genomes was of greater similarity to the 16S rRNA dataset. Particularly, selection was made of five random sets of Human Microbiome Project (HMP) (NCBI SRA ID: SRS058770, SRS063985, SRS016203, SRS062427, SRS052697) from both the 16S rRNA data and the shotgun data.

3-2: Taxonomic analysis

The taxonomic profiling for each shotgun dataset was calculated using the reference k-mer database of core genes in substantially the same manner as in Example 1 and the reference k-mer database of entire genomes in substantially the same manner as in Comparative Example 1. The 16S rRNA data is taxonomically profiled by the cloud platform EzBioCloud (www.ezbiocloud.net).

The accuracy of the reference k-mer database of core genes and the reference k-mer database of entire genomes was determined by 16S rRNA taxonomic profile prediction.

Tables 6-10 below show the total abundance of 16S rRNA and shotgun data for each HMP sample obtained in Example 3-1 at the genus level. In Tables 5 to 9, taxonomic profiling results obtained using data published to date are given in comparison with those in the 16S rRNA method, which has been most commonly used in taxonomic profiling. In Table 5 to Table 9, the taxonomic profiling results calculated using various published data are given, demonstrating that the method using the k-mer database of core genes according to the present invention has a high correlation with the existing method.

Among the five HMP samples, calculated taxonomic profiling results for NCBI SRA ID: SRS058770 are listed in Table 6, for NCBI SRA ID: RS063985 in Table 7, for NCBI SRA ID: SRS016203 in Table 8, for NCBI SRA ID: SRS062427 in Table 9, and for NCBI SRA ID: SRS052697 in Table 10.

[TABLE 6]

| Taxon name | 16S rRNA | Core gene K-mer | Full Genome K-mer |
|---|---|---|---|
| Bacteroides | 62.05% | 62.38% | 42.21% |
| Alistipes | 14.89% | 13.27% | 47.57% |
| Parabacteroides | 4.35% | 4.29% | 0.59% |
| Dialister | 1.86% | 1.67% | 0.80% |
| Oscillibacter | 1.69% | 1.80% | 0.18% |
| Odoribacter | 1.68% | 2.68% | 1.80% |
| Subdoligranulum | 1.61% | 1.48% | 0.67% |
| Roseburia | 1.44% | 0.96% | 0.51% |
| Faecalibacterium | 1.23% | 1.40% | 0.75% |
| Barnesiella | 1.16% | 1.07% | 0.69% |
| Parasutterella | 0.83% | 0.85% | 0.47% |
| Pseudoflavonifractor | 0.41% | 2.27% | 0.85% |

[TABLE 7]

| Taxon name | 16S rRNA | Core gene K-mer | Full Genome K-mer |
|---|---|---|---|
| Faecalibacterium | 59.05% | 60.53% | 43.04% |
| Bacteroides | 18.27% | 13.55% | 14.83% |
| Alistipes | 6.91% | 8.29% | 18.59% |
| Ruminococcus | 4.94% | 5.52% | 6.26% |
| Oscillibacter | 1.64% | 3.62% | 3.24% |
| Subdoligranulum | 1.14% | 0.64% | 0.75% |
| Parabacteroides | 1.12% | 1.69% | 0.17% |
| Lachnospira | 0.29% | 0.35% | 0.52% |
| Dialister | 0.21% | 1.88% | 2.50% |
| Paraprevotella | 0.18% | 0.53% | 0.11% |
| Odoribacter | 0.16% | 0.84% | 0.74% |
| Akkermansia | 0.15% | 2.49% | 2.86% |

[TABLE 8]

| Taxon name | 16S rRNA | Core gene K-me r | Full Genome K-m er |
|---|---|---|---|
| Bacteroides | 35.74% | 36.15% | 37.06% |
| Alistipes | 20.11 % | 20.87% | 31.84% |
| Faecalibacterium | 19.91% | 19.98% | 12.39% |
| Roseburia | 6.29% | 6.99% | 4.54% |

(continued)

| Taxon name | 16S rRNA | Core gene K-me r | Full Genome K-m er |
|---|---|---|---|
| Oscillibacter | 5.17% | 5.47% | 3.19% |
| Ruminococcus_g2 | 2.07% | 1.64% | 1.38% |
| Blautia | 2.04% | 0.34% | 0.35% |
| Parasutterella | 1.70% | 1.08% | 0.80% |
| Parabacteroides | 1.63% | 2.42% | 0.50% |
| Lachnospira | 1.32% | 0.87% | 0.76% |
| Subdoligranulum | 0.88% | 0.68% | 0.00% |
| Pseudoflavonifractor | 0.54% | 0.81% | 0.65% |
| Fusicatenibacter | 0.29% | 0.49% | 0.00% |
| Akkermansia | 0.21% | 0.92% | 0.84% |

[TABLE 9]

| Taxon name | 16S rRNA | Core gene K-mer | Full Genome K-mer |
|---|---|---|---|
| Bacteroides | 49.43% | 50.89% | 50.45% |
| Alistipes | 16.34% | 14.48% | 29.69% |
| Faecalibacterium | 12.73% | 6.55% | 2.96% |
| Parabacteroides | 7.73% | 7.49% | 0.71% |
| Roseburia | 2.80% | 1.41% | 0.90% |
| Subdoligranulum | 2.47% | 5.08% | 3.43% |
| Akkermansia | 0.97% | 8.95% | 6.84% |
| Fusicatenibacter | 0.44% | 0.76% | 0.00% |
| Oscillibacter | 0.35% | 0.83% | 0.60% |
| Ruminococcus | 0.35% | 0.66% | 0.46% |
| Blautia | 0.31% | 0.21% | 0.18% |
| Coprococcus_g2 | 0.16% | 0.30% | 0.00% |

[TABLE 10]

| Taxon name | 16S rRNA | Core gene K-mer | Full Genome K-mer |
|---|---|---|---|
| Bacteroides | 31.60% | 35.21% | 38.24% |
| Alistipes | 18.26% | 21.80% | 26.54% |
| Faecalibacterium | 11.58% | 10.62% | 7.33% |
| Oscillibacter | 5.32% | 5.02% | 3.09% |
| Subdoligranulum | 3.97% | 2.73% | 2.04% |
| Roseburia | 3.59% | 3.62% | 3.31% |
| Parabacteroides | 2.76% | 2.71% | 0.51% |
| Ruminococcus | 2.47% | 0.21% | 0.26% |
| Blautia | 2.44% | 0.28% | 0.40% |

(continued)

| Taxon name | 16S rRNA | Core gene K-mer | Full Genome K-mer |
|---|---|---|---|
| Lachnospira | 1.72% | 0.98% | 0.94% |
| Barnesiella | 1.40% | 1.98% | 2.53% |
| Dialister | 0.72% | 1.64% | 0.00% |

3-3: Bray-Curtis similarity analysis

For all HMP sets obtained in Example 3-1, Bray-Curtis similarity indices in the reference k-mer database of core genes according to Example 1, the reference k-mer database of entire genomes according to Comparative Example 1, and a 16S rRNA database for taxonomic classification were calculated in substantially the same manner as in Example 2-4 and are shown in Table 10, below.

Table 11 shows the Bray-Curtis similarity for all HMP sets using the three reference databases. In Table 11, the Bray-Curtis similarity index indicates similarity as it approaches zero(0) and dissimilarity similar as it approaches one(1).

[TABLE 11] Bray-Curtis similarity index

| **Classification method** | **Table** 5 | **Table** 6 | **Table** 7 | **Table** 8 | **Table** 9 |
|---|---|---|---|---|---|
| 16S <-> Core gene k-mer | 0.032364 | 0.084045 | 0.038592 | 0.120357 | 0.095052 |
| 16S <-> Full Genome k-mer | 0.328857 | 0.219903 | 0.162248 | 0.215257 | 0.191417 |
| Core gene k-mer <-> Full Genome k-mer | 0.343328 | 0.173016 | 0.146114 | 0.164038 | 0.108041 |

As is understood from the results of Bray-Curtis similarity analysis using the three databases for all the HMP sets in Table 11, the k-mer dataset of core genes according to Example 1 exhibits greater similarity to the 16S rRNA data, compared to the k-mer dataset of entire genomes according to Comparative Example 1.

## Claims

**1.** A method of identifying and classifying microorganisms in a sample, the method comprising the step of:

providing microbial genome information obtained from the sample;

obtaining a sample k-mer dataset using the microbial genome information; and
comparing the sample k-mer dataset with a microbial taxon information-assigned reference k-mer database of reference microbial core genes to identify and classify microbes in the sample.

2. The method of claim 1, wherein the microbial genome information is obtained by analysis using next generation sequencing (NGS).

3. The method of claim 1, wherein the step of obtaining a sample k-mer dataset is created by fragmenting individual sequencing reads obtained through next generation sequencing (NGS) into k-mer-long letter strings with the fragmenting site on each of the sequencing reads shifting by one base for each fragment.

4. The method of claim 3, wherein the length of k-mer is shorter than length of the sequencing reads.

5. The method of claim 1, wherein the length of the k-mers contained in the sample k-mer dataset and the reference k-mer database are the same.

6. The method of claim 1, wherein the reference k-mer database comprise at least one k-mer generated from each of the reference core genes and the k-mer is assigned with microbial taxon information.

7. The method of claim 1, wherein the taxon information is differentially assigned by a unique ID.

8. The method of claim 1, wherein the taxon information assigned to the k-mer contained in the reference k-mer database comprise information on a microbial species or the lowest common ancestor(LCA).

9. The method of claim 8, wherein the reference k-mer database of reference core genes is built by:

extracting sequence information of reference core genes from reference microbial genome information and dividing the sequence information of the reference core gene into k-mers to obtain one or more k-mers, and assigning a unique ID for microbial taxon information to each of the k-mers
wherein the assigning a unique ID for taxon information to each of the k-mers is carried out by

(i) assigning unique ID of the microbial species to which the k-mers belong, when the k-mers are unique k-mers,
(ii) assigning unique ID of the microbial species when the k-mers are distinct k-mers and are found only in one microbial species, or
(iii) assigning unique ID of the microbial taxon information with selecting the lowest common ancestor(LCA), when the k-mers are distinct k-mers and are found in various microbial species.

10. The method of claim 1, wherein the comparing the sample k-mer dataset with a reference k-mer database is performed by selecting a k-mer being exactly identical in nucleic acid sequence information contained in the reference k-mer database of reference core genes, with the k-mer contained in a sample k-mer dataset, and listing indices accounting for taxon information of the selected k-mer.

11. The method of claim 1, wherein the comparing the sample k-mer dataset with a reference k-mer database of core genes is carried out using a KRAKEN program.

12. The method of claim 1, wherein,
the microbial taxon information is classified by unique ID values and is assigned to individual k-mers in the reference k-mer database,
the sample microbial genome information includes sequencing reads obtained by next generation sequencing (NGS), and
for individual sequencing reads of the sample microbial genome,

(i) creating a k-mer dataset including one or more k-mers and comparing with the reference k-mer database of reference microbial core genes to select a k-mer whose nucleotide sequence is exactly matched, from the reference k-mer database,
(ii) obtaining unique ID information assigned to the selected k-mer,
(iii) selecting an ID as a unique ID for a sequencing read if a unique ID list includes one unique ID or all identical unique IDs, or selecting a unique ID corresponding to the least common ancestor (LCA) if a unique ID list includes two or more different unique IDs, using list information including one or more unique IDs obtained for the sequencing reads,
(iv) combining taxon information of the unique IDs corresponding to the taxonomic levels assigned to individual sequencing reads, and

the microbe in the sample is identified and classified by generating a full unique ID list with collecting the unique IDs corresponding to the taxonomic levels obtained for the individual sequencing reads for entire sequencing reads included in the sample microbial genome.

**13.** A method for obtaining abundance profiling information of microbial species in a sample, the method comprising the steps of:

providing microbial genome information obtained from the sample;
obtaining a sample k-mer dataset using the microbial genome information; and
comparing the sample k-mer dataset with a microbial taxon information-assigned reference k-mer database of reference microbial core genes,
wherein the microbial taxon information is classified by unique ID values and is assigned to individual k-mers in the reference k-mer database,
wherein the sample microbial genome information includes sequencing reads obtained by next generation sequencing (NGS), and
for individual sequencing reads of the sample microbial genome,

(i) creating a k-mer dataset including one or more k-mers and comparing with the reference k-mer database of reference microbial core genes to select a k-mer whose nucleotide sequence is exactly matched, from the reference k-mer database,
(ii) obtaining unique ID information assigned to the selected k-mer,
(iii) selecting an ID as a unique ID for a sequencing read if a unique ID list includes one unique ID or all identical unique IDs, or selecting a unique ID corresponding to the least common ancestor (LCA) if a unique ID list includes two or more different unique IDs, using list information including one or more unique IDs obtained for the sequencing reads,
(iv) combining taxon information of the unique IDs corresponding to the taxonomic levels assigned to individual sequencing reads, and

generating an entire unique ID list with collecting the unique IDs corresponding to the taxonomic levels obtained for the individual sequencing reads for entire sequencing reads included in the sample microbial genome,
obtaining the number of the classified reads by unique ID corresponding to the taxonomic level, from the full unique ID list for microbes in the sample, and
obtaining an abundance in the sample for the microbial species or taxon information corresponding to the unique IDs by dividing the number of the classified reads by unique ID with a sum of the number of classified reads in the full unique ID list.

**14.** A system of identifying and classifying a microorganism in a sample, the system comprising: (a) a reference k-mer database of reference microbial core genes; and (b) a processor equipped with a k-mer extractor and a k-mer analyzer,
wherein the reference k-mer database comprises at least one k-mer generated from DNA information of at least one reference microbial core gene, and the k-mer is assigned with microbial taxon information,
wherein the k-mer extractor in the processor extracts at least one k-mer from metagenomic information obtained from the sample to generate k-mer dataset; and
wherein the k-mer analyzer in the processor selects a k-mer exactly identical in nucleic acid sequence information from the k-mers contained in the reference k-mer database of reference core genes with respect to the k-mer contained in a sample k-mer dataset, lists unique IDs accounting for taxon information of the selected k-mer, and identifies and classifies the microbe in the sample, based on the taxonomic information about the selected k-mer.

Fig. 1

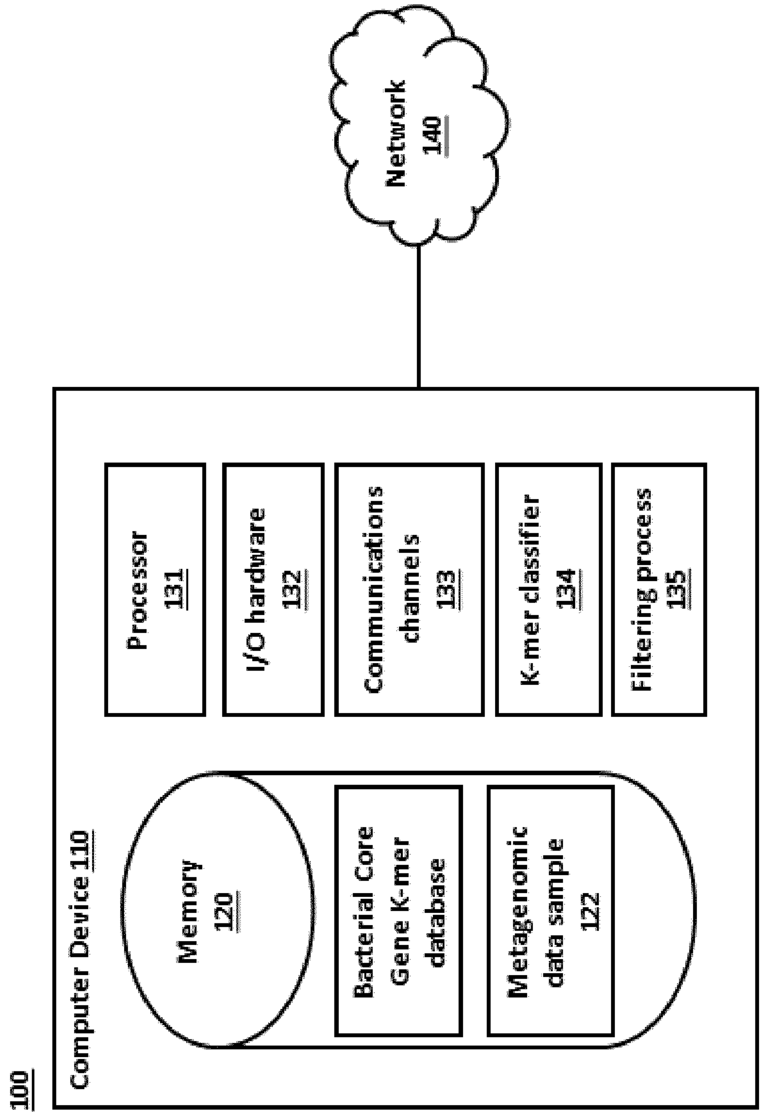
Network
140
Computer Device 110
100
Memory
120
Bacterial Core Gene K-mer database
Metagenomic data sample
122
Processor
131
I/O hardware
132
Communications channels
133
K-mer classifier
134
Filtering process
135

Fig. 2

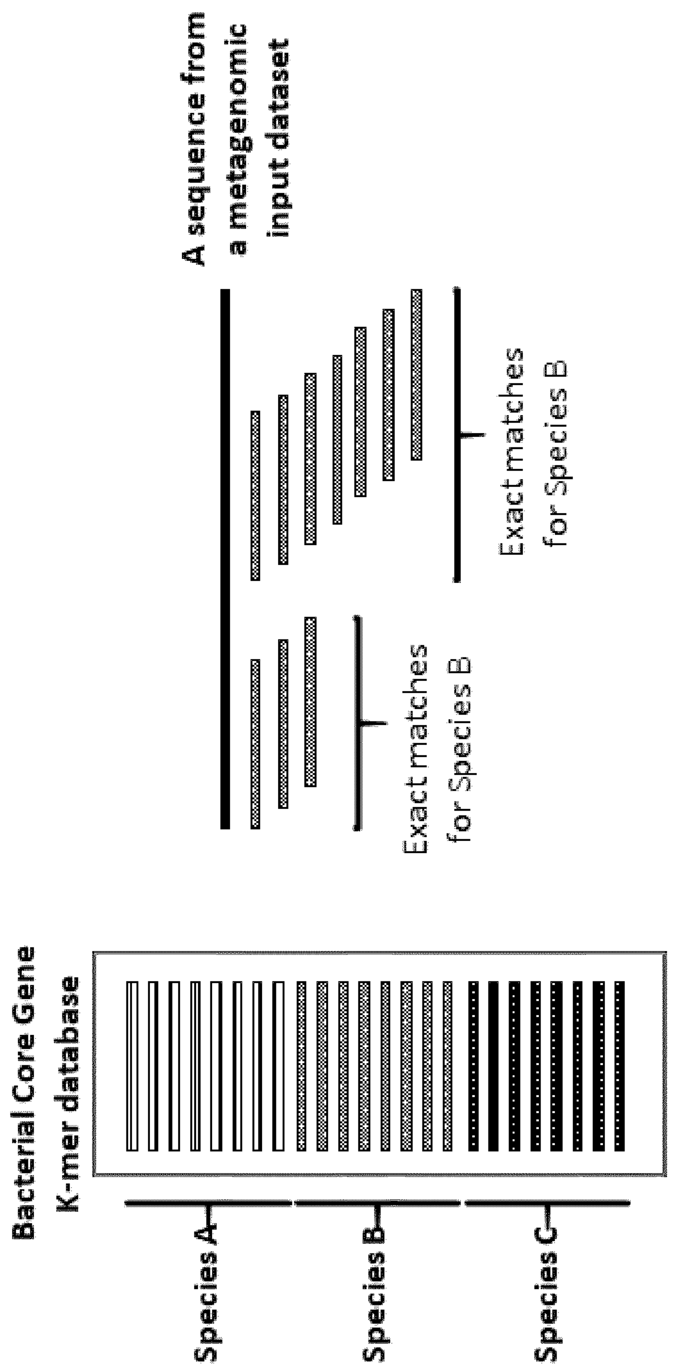
A sequence from
a metagenomic
input dataset
Exact matches
for Species B
Exact matches
for Species B
Bacterial Core Gene
K-mer database
Species A
Species B
Species C

Fig. 3

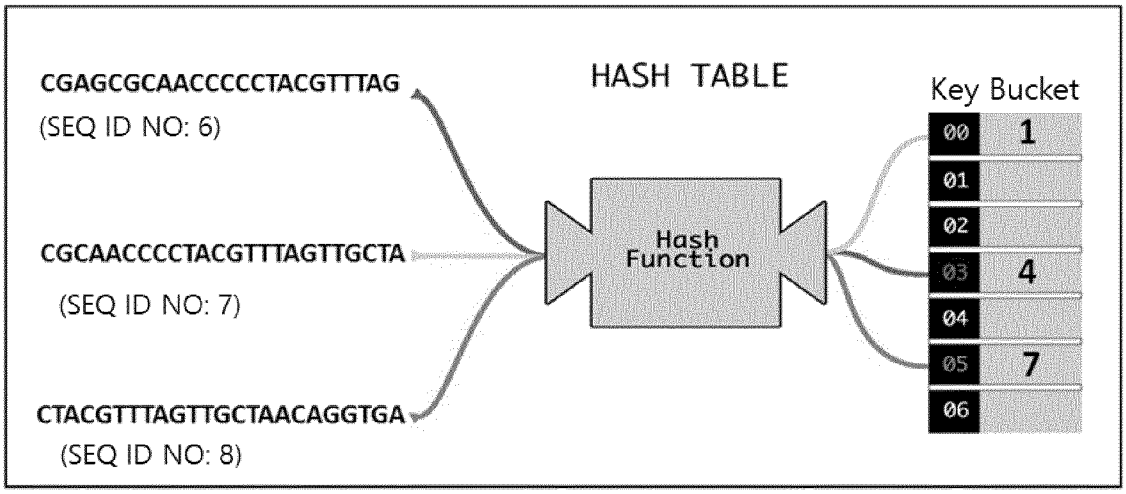
CGAGCGCAACCCCCTACGTTTAG
(SEQ ID NO: 6)
CGCAACCCCTACGTTTAGTTGCTA
(SEQ ID NO: 7)
CTACGTTTAGTTGCTAACAGGTGA
(SEQ ID NO: 8)
HASH TABLE
Hash Function
Key Bucket
00
1
01
02
03
4
04
05
7
06

Fig. 4

Hash table

| Key | Value |
|---|---|
| CGAGCGCAACCC (SEQ ID NO: 2) | 5756 |
| GGAGCGCAACCC (SEQ ID NO: 3) | 2347 |

Taxonomy tree

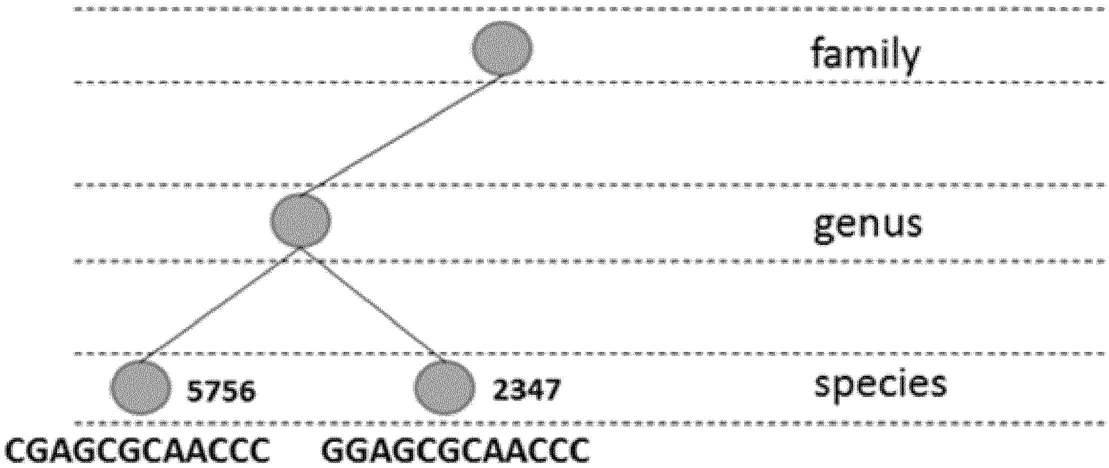

Fig. 5

**Hash table**

| Key | Value |
|---|---|
| CGAGCGCAACCC (SEQ ID NO: 2) | 930 |
| GGAGCGCAACCC (SEQ ID NO: 3) | 2347 |

**Taxonomy tree**

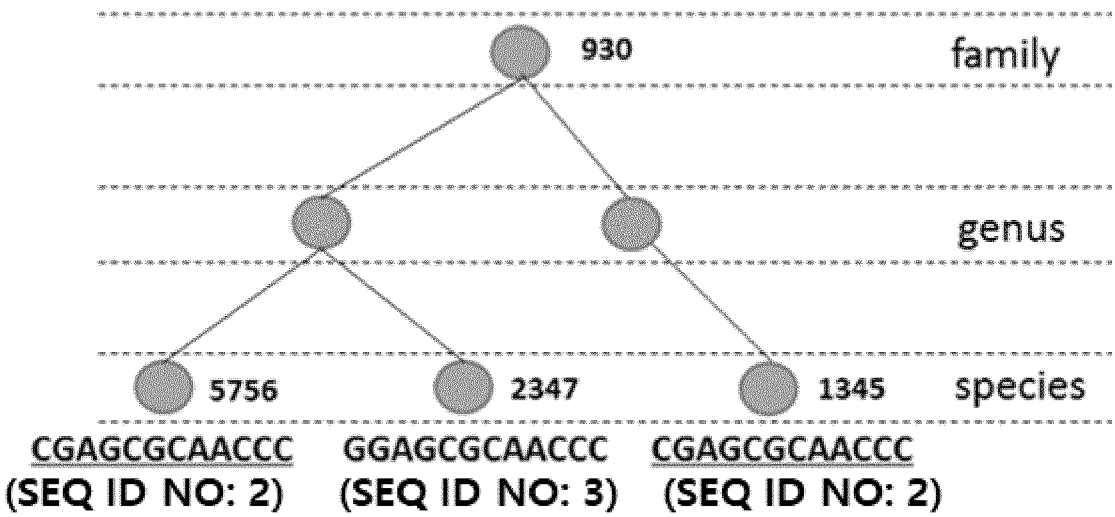

Fig. 6

## Hash table in memory

| Key | Value |
| --- | --- |
| CGAGCGCAACCC (SEQ ID NO: 2) | 5756 |
| GGAGCGCAACCC (SEQ ID NO: 3) | 2347 |
| AGCGCAACCCGT (SEQ ID NO: 4) | 1345 |
| GCGCAACCCGTT (SEQ ID NO: 5) | 1345 |

## Taxonomy tree

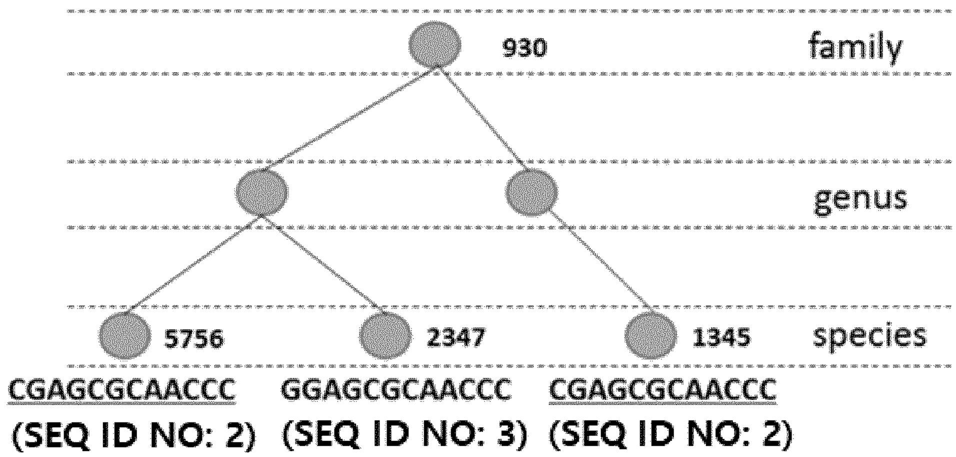


Query read: CGAGCGCAACCCGTT (SEQ ID NO: 1)

K-mer classification: 5756, 2347, 1345, 1345

Read classification: 930

**INTERNATIONAL SEARCH REPORT**

International application No.
**PCT/KR2019/011410**

A. CLASSIFICATION OF SUBJECT MATTER

*G16B 40/00(2019.01)i, G16B 30/10(2019.01)i*

According to International Patent Classification (IPC) or to both national classification and IPC

B. FIELDS SEARCHED

Minimum documentation searched (classification system followed by classification symbols)
G16B 40/00; C12Q 1/68; G06F 19/22; G16B 30/10

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: extract k-mer matching method, bacterial core genes, taxon

C. DOCUMENTS CONSIDERED TO BE RELEVANT

| Category* | Citation of document, with indication, where appropriate, of the relevant passages | Relevant to claim No. |
|---|---|---|
| Y | KAWULOK, J. et al. CoMeta: classification of metagenomes using k-mers. PloS One. 17 April 2015, vol. 10, no. 4, e0121453 (pages 1-23) See abstract; page 2, Metagenomic processing section; page 3, third paragraph; page 4, third paragraph; page 5, second paragraph-page 10, first paragraph; figure 1. | 1-8,10,11,14 |
| A | | 9,12,13 |
| Y | NA, S.-I. et al. UBCG: Up-to-date bacterial core gene set and pipeline for phylogenomic tree reconstruction. Journal of Microbiology. 2018, vol. 56, no. 4, pages 280-285, (electronic publication) 28 February 2018 See abstract; page 280, right column. | 1-8,10,11,14 |
| A | WANG, Y. et al. Identifying group-specific sequences for microbial communities using long k-mer sequence signatures. Frontiers in Microbiology. 03 May 2018, vol. 9, article 872 (pages 1-18) See the entire document. | 1-14 |
| A | RAIME, K. et al. Method for the identification of taxon-specific k-mers from chloroplast genome: a case study on tomato plant (Solanum lycopersicum). Frontiers in Plant Science. 17 January 2018, vol. 9, article 6 (pages 1-12) See the entire document. | 1-14 |

☒ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 06 DECEMBER 2019 (06.12.2019) | **06 DECEMBER 2019 (06.12.2019)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

INTERNATIONAL SEARCH REPORT

International application No.
PCT/KR2019/011410

C (Continuation). DOCUMENTS CONSIDERED TO BE RELEVANT

| Category* | Citation of document, with indication, where appropriate, of the relevant passages | Relevant to claim No. |
|---|---|---|
| A | KR 10-1798229 B1 (CHUNLAB, INC.) 12 December 2017<br>See the entire document. | 1-14 |
| A | WO 2016-172643 A2 (UNIVERSITY OF UTAH RESEARCH FOUNDATION)<br>27 October 2016<br>See the entire document. | 1-14 |

Form PCT/ISA/210 (continuation of second sheet) (January 2015)

**INTERNATIONAL SEARCH REPORT**
Information on patent family members

International application No.
**PCT/KR2019/011410**

| Patent document cited in search report | Publication date | Patent family member | Publication date |
|---|---|---|---|
| KR 10-1798229 B1 | 12/12/2017 | EP 3511847 A1 | 17/07/2019 |
| | | US 2019-0119717 A1 | 25/04/2019 |
| | | WO 2018-124661 A1 | 05/07/2018 |
| WO 2016-172643 A2 | 27/10/2016 | AU 2016-253004 A1 | 07/09/2017 |
| | | CA 2977548 A1 | 27/10/2016 |
| | | CN 107532332 A | 02/01/2018 |
| | | EP 3286359 A2 | 28/02/2018 |
| | | IL 254094 A | 31/10/2017 |
| | | US 2018-0365375 A1 | 20/12/2018 |
| | | WO 2016-172643 A3 | 09/02/2017 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SEONG-IN NA et al.** *Journal of Microbiology,* 2018, vol. 56 (4), 280-285
- **LASKAR F et al.** Diversity of methanogenic archaea in freshwater sediments of lacustrine ecosystems. *J Basic Microbiol,* February 2018, vol. 58 (2), 101-119